(19) Europäisches Patentamt
European Patent Office
Office européen des brevets

(11) Veröffentlichungsnummer: **0 622 364 A2**

# (12) EUROPÄISCHE PATENTANMELDUNG

(21) Anmeldenummer: **94105774.7**

(22) Anmeldetag: **14.04.94**

(51) Int. Cl.[5]: **C07D 401/04**, C07D 211/90, C07D 215/12, A61K 31/445

(30) Priorität: **27.04.93 DE 4313691**

(43) Veröffentlichungstag der Anmeldung: **02.11.94 Patentblatt 94/44**

(84) Benannte Vertragsstaaten: **AT BE CH DE DK ES FR GB GR IE IT LI LU MC NL PT SE**

(71) Anmelder: **BAYER AG**

**D-51368 Leverkusen (DE)**

(72) Erfinder: **Stoltefuss, Jürgen, Dipl.-Ing.**
**Parkstrasse 20**
**D-42781 Haan (DE)**
Erfinder: **Goldmann, Siegfried, Dr.**
**Am Osterholz 91**
**D-42327 Wuppertal (DE)**
Erfinder: **Straub, Alexander, Dr.**
**Moospfad 30**
**D-42113 Wuppertal (DE)**
Erfinder: **Bechem, Martin, Dr.**
**Hans-Böckler-Strasse 102**
**D-42111 Wuppertal (DE)**
Erfinder: **Gross, Rainer, Dr.**
**Platzhofstrasse 23**
**D-42115 Wuppertal (DE)**
Erfinder: **Hebisch, Siegbert, Dr.**
**Johann-Breuker-Platz 8**
**D-46244 Bottrop (DE)**
Erfinder: **Hütter, Joachim, Dr.**
**Teschen-Sudberger-Strasse 13**
**D-42349 Wuppertal (DE)**
Erfinder: **Rounding, Howard-Paul, Dr.**
**Pahlkestrasse 15**
**D-42115 Wuppertal (DE)**

## (54) 2,6-Disubstituierte 4-Chinolyldihydropyridine.

(57) Die Erfindung betrifft neue 2,6-Disubstituierte 4-Chinolyl-dihydropyridine

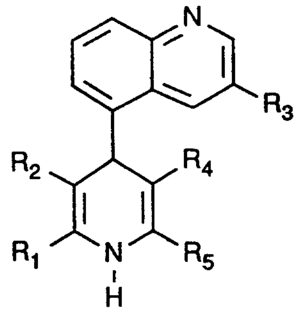

(I),

in welcher

$R_1$ bis $R_5$ die in der Beschreibung angegebene Bedeutung haben,

Verfahren zu ihrer Herstellung und ihre Verwendung in Arzneimitteln, insbesondere in Mitteln zur Behandlung von Herz-Kreislauf-Erkrankungen.

EP 0 622 364 A2

Die Erfindung betrifft neue 2,6-Disubstituierte 4-Chinolyl-dihydropyridine, Verfahren zu ihrer Herstellung und ihre Verwendung in Arzneimitteln, insbesondere in Mitteln zur Behandlung von Herz-Kreislauferkrankungen.

Es ist bereits bekannt, daß 1,4-Dihydropyridine gefäßerweiternde Eigenschaften besitzen und als Coronarmittel und Antihypertensiva verwendet werden können. Weiterhin ist bekannt, daß 1,4-Dihydropyridine eine Hemmung der Kontraktionskraft von glatten und cardialen Muskeln bewirken und zur Behandlung von Coronar- und Gefäßerkrankungen eingesetzt werden können. Aus US 5 100 900 sind auch bereits 4-Chinolyl-dihydropyridine bekannt, die eine positiv isotrope Wirkung besitzen.

Bei Kenntnis des Standes der Technik war es nicht vorhersehbar, daß die erfindungsgemäßen Verbindungen die Kontraktionskraft des Herzens beeinflussen und deshalb zur Bekämpfung von cardiovaskulären Erkrankungen verwendet werden können.

Die vorliegende Erfindung betrifft neue 2,6-Disubstituierte 4-Chinolyl-dihydropyridine der allgemeinen Formel (I)

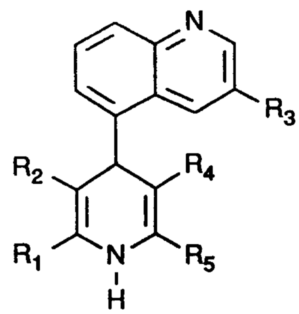

(I)

in welcher

$R^1$ und $R^5$ gleich oder verschieden sind und für Wasserstoff, Cyano, Formyl, Trifluormethyl oder für geradkettiges oder verzweigtes Alkyl mit bis zu 8 Kohlenstoffatomen stehen, das gegebenenfalls durch Hydroxy, geradkettiges oder verzweigtes Alkoxy oder Alkoxycarbonyl mit jeweils bis zu 8 Kohlenstoffatomen oder durch eine Gruppe der Formel $-NR^6R^7$, $-O-CO-R^8$, $-O-(CH_2)_a-OR^{8'}$ oder $-O-(CH_2)_{a'}-NR^9R^{10}$ substituiert ist,

worin

$R^6$, $R^7$, $R^9$ und $R^{10}$ gleich oder verschieden sind und Wasserstoff, Phenyl oder geradkettiges oder verzweigtes Alkyl mit bis zu 6 Kohlenstoffatomen bedeuten,

$R^8$ und $R^{8'}$ gleich oder verschieden sind und geradkettiges oder verzweigtes Alkyl mit bis zu 6 Kohlenstoffatomen bedeuten,

und

a und a' gleich oder verschieden sind und eine Zahl 2, 3, 4 oder 5 bedeuten,

$R^2$ für Nitro, Cyano oder Formyl steht,

oder

$R^1$ und $R^2$ gemeinsam einen Lactonring der Formel

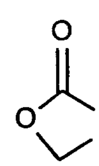

bilden,

$R^3$ für Aryl mit 6 bis 10 Kohlenstoffatomen steht, das gegebenenfalls bis zu 3-fach gleich oder verschieden durch Halogen, Nitro, Cyano, Hydroxy, Amino, Trifluormethyl, Trifluormethoxy, Trifluormethylthio, geradkettiges oder verzweigtes Alkyl, Alkoxy oder Alkoxycarbonyl mit jeweils bis zu 8 Kohlenstoffatomen oder Carboxy substituiert ist, oder

für Thienyl oder Pyridyl steht, die gegebenenfalls durch Halogen substituiert

sind,

$R^4$ für eine Gruppe der Formel $-CO-NR^{12}R^{13}$, $-CO-A-R^{14}$ oder $-P(O)(OR^{15})(OR^{16})$ steht,

worin

$R^{12}$ und $R^{13}$ gleich oder verschieden sind und Wasserstoff oder einen geradkettigen, verzweigten, cyclischen, gesättigten oder ungesättigten Kohlenwasserstoffrest mit bis zu 10 Kohlenstoffatomen bedeuten, der gegebenenfalls durch Cycloalkyl mit 3 bis 8 Kohlenstoffatomen, Halogen, Hydroxy, Cyano oder durch Aryl, Aryloxy, Arylthio mit jeweils 6 bis 10 Kohlenstoffatomen oder durch einen 5- bis 7-gliedrigen, gesättigen oder ungesättigten Heterocyclus mit bis zu 3 Heteroatomen aus der Reihe S, N oder O substituiert ist, wobei die Cyclen ihrerseits durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert sein können, oder

Aryl mit 6 bis 10 Kohlenstoffatomen oder einen 5- bis 7-gliedrigen, gesättigten oder ungesättigten Heterocyclus mit bis zu 3 Heteroatomen aus der Reihe S, N oder O bedeuten, die gegebenenfalls bis zu 2-fach gleich oder verschieden durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert sind,

oder

$R^{12}$ und $R^{13}$ gemeinsam unter Einbezug des Stickstoffatoms einen 5- bis 8-gliedrigen, gesättigen oder ungesättigten Heterocyclus bilden, der gegebenenfalls durch Sauerstoff oder durch Formel $S(O)_b$, -CO- oder $-NR^{17}$ unterbrochen ist,

worin

b eine Zahl 0, 1 oder 2 bedeutet,

$R^{17}$ Wasserstoff oder Aryl mit 6 bis 10 Kohlenstoffatomen bedeutet, das gegebenenfalls bis zu 2-fach gleich oder verschieden durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert ist, oder

einen cyclischen, geradkettigen oder verzweigten, gesättigten oder ungesättigten Kohlenwasserstoffrest mit bis zu 8 Kohlenstoffatomen bedeutet, der gegebenenfalls durch Hydroxy, Halogen oder durch Aryl mit 6 bis 10 Kohlenstoffatomen oder einen 5- bis 7-gliedrigen, gesättigten oder ungesättigten Heterocyclus mit 3 Heteroatomen aus der Reihe S, N oder O substituiert ist, die ihrerseits bis zu 2-fach gleich oder verschieden durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert sein können,

und der Heterocyclus gegebenenfalls durch geradkettiges oder verzweigtes Alkoxy oder Alkylthio mit jeweils bis zu 4 Kohlenstoffatomen, Halogen, Ary lmit 6 bis 10 Kohlenstoffatomen, einen 4- bis 7-gliedrigen, gesättigten oder ungesättigten Heterocyclus mit bis zu 3 Heteroatomen aus der Reihe S, N oder O oder durch geradkettiges oder verzweigtes Alkyl mit bis zu 4 Kohlenstoffatomen substituiert ist, das gegebenenfalls seinerseits durch Aryl mit 6 bis 10 Kohlenstoffatomen substituiert sein kann,

A eine direkte Bindung oder ein Sauerstoffatom bedeutet,

$R^{14}$ Wasserstoff oder Aryl mit 6 bis 10 Kohlenstoffatomen oder einen 4- bis 7-gliedrigen, gesättigten oder ungesättigten Heterocyclus mit bis zu 3 Heteroatomen aus der Reihe S, N oder O bedeutet, die gegebenenfalls bis zu 3-fach gleich oder verschieden durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert sind, oder

einen cyclischen, geradkettigen oder verzweigten, gesättigten oder ungesättigten Kohlenwasserstoffrest mit bis zu 12 Kohlenstoffatomen bedeutet, der gegebenenfalls bis zu 3-fach gleich oder verschieden durch Sauerstoff -CO-, -CO-NH-, -O-CO-, -CO-O-, -NH-CO-, $-SO_2-NH-$, $-NH-SO_2-$, $-S(O)_c-$oder $-NR^{18}$ unterbrochen ist,

worin

c — die oben angegebene Bedeutung von b hat und mit dieser gleich oder verschieden ist,

$R^{18}$ — die oben angegebene Bedeutung von $R^{17}$ hat und mit dieser gleich oder verschieden ist,

oder der bis zu 3-fach gleich oder verschieden unterbrochen ist durch Aryliden mit 6 bis 10 Kohlenstoffatomen oder einen cyclischen Rest der Formel

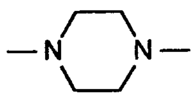 , 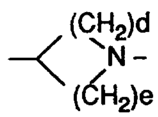 , 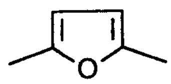 oder 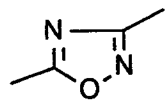

worin

d und e — gleich oder verschieden sind und eine Zahl 1 oder 2 bedeuten,

und worin Aryliden und die Heterocyclen ihrerseits durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert sein können,

und wobei der Kohlenwasserstoffrest gegebenenfalls bis zu 3-fach gleich oder verschieden durch Cycloalkyl mit 3 bis 8 Kohlenstoffatomen, Halogen, Nitro, Cyano, Hydroxy, $-O-NO_2$, geradkettiges oder verzweigtes Alkylthio, Alkoxy oder Acyloxy mit jeweils bis zu 8 Kohlenstoffatomen oder durch Aryl, Aryloxy oder Arylthio mit jeweils 6 bis 10 Kohlenstoffatomen oder durch einen 5- bis 7-gliedrigen, gesättigten oder ungesättigen Heterocyclus mit bis zu 3 Heteroatomen aus der Reihe S, N oder O substituiert ist, wobei die Cyclen ihrerseits bis zu 3-fach gleich oder verschieden durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert sein können, oder

der Kohlenwasserstoffrest durch eine Gruppe der Formel $-CO_2-R^{19}$, $-CONR^{20}R^{21}$ oder $-NR^{22}R^{23}$ substituiert ist,

worin

$R^{19}$ — die oben angegebene Bedeutung von $R^{17}$ hat und mit dieser gleich oder verschieden ist

und

$R^{20}$, $R^{21}$, $R^{22}$ und $R^{23}$ — die oben angegebene Bedeutung von $R^{12}$ und $R^{13}$ haben und mit diesen gleich oder verschieden sind,

$R^{15}$ und $R^{16}$ — gleich oder verschieden sind und geradkettiges oder verzweigtes Alkyl mit bis zu 8 Kohlenstoffatomen bedeuten,

oder

$R^{15}$ und $R^{16}$ — gemeinsam unter Einbezug der Sauerstoffatome und des Phosphors einen 5- bis 7-gliedrigen, gesättigen Heterocyclus bilden,

und deren Salze,

mit der Maßgabe, daß $R^5$ nicht für unsubstituiertes Alkyl stehen darf, wenn $R^1$ gleichzeitig unsubstituiertes Alkyl bedeutet oder $R^1$ und $R^2$ gemeinsam einen Lactonring bilden.

Physiologisch unbedenkliche Salze können Salze der erfindungsgemäßen Verbindungen mit anorganischen oder organischen Säuren sein. Bevorzugt werden Salze mit anorganischen Säuren wie beispielsweise Salzsäure, Bromwasserstoffsäure, Phosphorsäure oder Schwefelsäure, oder Salze mit organischen Carbon- oder Sulfonsäuren wie beispielsweise Essigsäure, Maleinsäure, Fumarsäure, Äpfelsäure, Zitronensäure, Weinsäure, Milchsäure, Benzoesäure, oder Methansulfonsäure, Ethansulfonsäure, Phenylsulfonsäure, Toluolsulfonsäure oder Naphthalindisulfonsäure.

Die erfindungsgemäßen Verbindungen existieren in stereoisomeren Formen, die sich entweder wie Bild und Spiegelbild (Enantiomere), oder die sich nicht wie Bild und Spiegelbild (Diastereomere) verhalten. Die Erfindung betrifft sowohl die Antipoden als auch die Racemformen sowie die Diastereomerengemische. Die Racemformen lassen sich ebenso wie die Diastereomeren in bekannter Weise in die stereoisomer

einheitlichen Bestandteile trennen.

Bevorzugt sind Verbindungen der allgemeinen Formel (I),
in welcher

$R^1$ und $R^5$ gleich oder verschieden sind und für Wasserstoff, Cyano, Formyl, Trifluormethyl oder für geradkettiges oder verzweigtes Alkyl mit bis zu 6 Kohlenstoffatomen stehen, das gegebenenfalls durch Hydroxy, geradkettiges oder verzweigtes Alkoxy oder Alkoxycarbonyl mit jeweils bis zu 4 Kohlenstoffatomen oder durch eine Gruppe der Formel $-NR^6R^7$, $-O-CO-R^8$, $-O-(CH_2)_a-OR^{8'}$ oder $-O-(CH_2)_{a'}-NR^9R^{10}$ substituiert ist,
worin

$R^6$, $R^7$, $R^9$ und $R^{10}$ gleich oder verschieden sind und Wasserstoff, Phenyl oder geradkettiges oder verzweigtes Alkyl mit bis zu 4 Kohlenstoffatomen bedeuten,

$R^8$ und $R^{8'}$ gleich oder verschieden sind und geradkettiges oder verzweigtes Alkyl mit bis zu 4 Kohlenstoffatomen bedeuten,
und

a und a' gleich oder verschieden sind und eine Zahl 2, 3 oder 4 bedeuten,

$R^2$ für Nitro, Cyano oder Formyl steht,

oder

$R^1$ und $R^2$ gemeinsam einen Lactonring der Formel

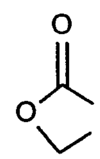

bilden,

$R^3$ für Phenyl steht, das gegebenenfalls durch Fluor, Chlor, Nitro, Cyano, Hydroxy, Amino, Trifluormethyl oder durch geradkettiges oder verzweigtes Alkyl oder Alkoxy mit jeweils bis zu 4 Kohlenstoffatomen substituiert ist, oder
für Thienyl oder Pyridyl steht, die gegebenenfalls durch Fluor, Chlor oder Brom substituiert sind,

$R^4$ für eine Gruppe der Formel $-CO-NR^{12}R^{13}$, $-CO-A-R^{14}$ oder $-P(O)(OR^{15})(OR^{16})$ steht,
worin

$R^{12}$ und $R^{13}$ gleich oder verschieden sind und Wasserstoff oder einen geradkettigen, verzweigten, cyclischen, gesättigten oder ungesättigten Kohlenwasserstoffrest mit bis zu 6 Kohlenstoffatomen bedeuten, der gegebenenfalls durch Cyclopropyl, Cyclopentyl, Cyclohexyl, Fluor, Chlor, Brom, Hydroxy oder durch Phenyl oder Pyridyl substituiert ist, wobei die Cyclen ihrerseits durch Fluor, Chlor, Brom, Cyano oder durch geradkettiges oder verzweigtes Alkyl, Alkoxy, Alkylthio, Alkoxycarbonyl mit jeweils bis zu 3 Kohlenstoffatomen, Trifluormethyl oder Trifluormethoxy substituiert sein können, oder
Phenyl oder Pyridyl bedeuten, die gegebenenfalls durch Fluor, Chlor oder durch geradkettiges oder verzweigtes Alkyl, Alkoxy, Alkylthio, Alkoxycarbonyl mit jeweils bis zu 4 Kohlenstoffatomen, Trifluormethyl oder Trifluormethoxy substituiert sind,
oder

$R^{12}$ und $R^{13}$ gemeinsam unter Einbezug des Stickstoffatoms einen 5- bis 7-gliedrigen, gesättigten oder ungesättigten Heterocyclus bilden, der gegebenenfalls durch Sauerstoff oder durch $S(O)_b$, -CO- oder $-NR^{17}$ unterbrochen sein kann,
worin

b eine Zahl 0, 1 oder 2 bedeutet,

$R^{17}$ Wasserstoff oder Phenyl bedeutet, oder
einen cyclischen, geradkettigen oder verzweigten, gesättigten oder ungesättigten Kohlenwasserstoffrest mit bis zu 6 Kohlenstoffatomen bedeutet, der gegebenenfalls durch Fluor, Chlor, Brom oder durch Phenyl oder Pyridyl substituiert

ist, die ihrerseits durch Fluor, Chlor, Methyl, Ethyl, Methoxy, Ethoxy, Trifluormethyl oder Trifluormethoxy substituiert sein können,

und der Heterocyclus gegebenenfalls durch geradkettiges oder verzweigtes Alkxoy oder Alkylthio mit jeweils bis zu 3 Kohlenstoffatomen, Fluor, Chlor, Brom, Phenyl oder Pyridyl oder

durch verzweigtes Alkyl mit bis zu 4 Kohlenstoffatomen oder Benzyl substituiert ist,

A eine direkte Bindung oder ein Sauerstoffatom bedeutet,

$R^{14}$ Wasserstoff, Phenyl oder Pyridyl bedeutet, die gegebenenfalls durch Fluor, Chlor oder durch geradkettiges oder verzweigtes Alkyl, Alkoxy oder Alkylthio mit jeweils bis zu 3 Kohlenstoffatomen, Trifluormethyl oder Trifluormethoxy substituiert sind, oder

einen cyclischen, geradkettigen oder verzweigten, gesättigten oder ungesättigten Kohlenwasserstoffrest mit bis zu 10 Kohlenstoffatomen bedeutet, der gegebenenfalls bis zu 2-fach gleich oder verschieden durch Sauerstoff oder durch -CO-, -CO-NH-, -O-CO-, -CO-O-, -NH-CO-, $-SO_2-NH-$, $-NH-SO_2-$, $-S(O)_c-$oder $NR^{18}$ unterbrochen ist,

worin

c die oben angegebene Bedeutung von b hat und mit dieser gleich oder verschieden ist,

$R^{18}$ die oben angegebene Bedeutung von $R^{17}$ hat und mit dieser gleich oder verschieden ist,

oder der bis zu 2-fach gleich oder verschieden unterbrochen ist durch Phenyliden oder einen cyclischen Rest der Formel

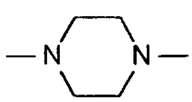 , 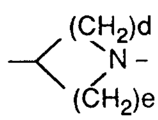 , 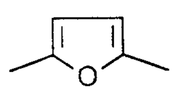 oder 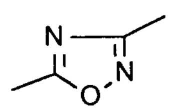

worin

d und e gleich oder verschieden sind und eine Zahl 1 oder 2 bedeuten,

und wobei der Kohlenwasserstoffrest gegebenenfalls bis zu 2-fach gleich oder verschieden durch Cyclopropyl, Cyclobutyl, Cyclopentyl, Cyclohexyl, Fluor, Chlor, Brom, Nitro, Cyano, Hydroxy, $-O-NO_2$, geradkettiges oder verzweigtes Alkylthio, Alkoxy oder Acyloxy mit jeweils bis zu 6 Kohlenstoffatomen oder durch Phenyl, Phenoxy, Phenylthio oder Pyridyl substituiert ist, wobei die Cyclen ihrerseits bis zu 2-fach gleich oder verschieden durch Fluor, Chlor, Brom, Cyano oder durch geradkettiges oder verzweigtes Alkyl, Alkoxy, Alkylthio, Alkoxycarbonyl mit jeweils mit bis zu 4 Kohlenstoffatomen, Trifluormethyl oder Trifluormethoxy substituiert sind, oder

der Kohlenwasserstoffrest durch eine Gruppe der Formel $-CO_2-R^{19}$, $-CONR^{20}R^{21}$ oder $-NR^{22}R^{23}$ substituiert ist,

worin

$R^{19}$ die oben angegebene Bedeutung von $R^{17}$ hat und mit dieser gleich oder verschieden ist

und

$R^{20}$, $R^{21}$, $R^{22}$ und $R^{23}$ die oben angegebene Bedeutung von $R^{12}$ und $R^{13}$ haben und mit diesen gleich oder verschieden sind,

$R^{15}$ und $R^{16}$ gleich oder verschieden sind und geradkettiges oder verzweigtes Alkyl mit bis zu 6 Kohlenstoffatomen bedeuten

oder

$R^{15}$ und $R^{16}$ gemeinsam unter Einbezug der Sauerstoffatome und des Phosphors einen 6-gliedrigen gesättigten Cyclus bilden

und deren Salze,

mit der Maßgabe, daß $R^5$ nicht für unsubstituiertes Alkyl stehen darf, wenn $R^1$ gleichzeitig unsubstituiertes Alkyl bedeutet oder $R^1$ und $R^2$ gemeinsam einen Lactonring bilden.

Besonders bevorzugt sind Verbindungen der allgemeinen Formel (I),
in welcher

| | |
|---|---|
| $R^1$ und $R^5$ | gleich oder verschieden sind und für Wasserstoff, Cyano, Formyl, Trifluormethyl oder Methyl oder Ethyl stehen, die gegebenenfalls durch Hydroxy, geradkettiges oder verzweigtes Alkoxy oder Alkoxycarbonyl mit jeweils bis zu 4 Kohlenstoffatomen oder durch eine Gruppe der Formel $-NR^6R^7$, $-O-CO-R^8$, $-O-(CH_2)_a-OR^{8'}$ oder $-O-(CH_2)_{a'}-NR^9R^{10}$ substituiert sind, worin |
| $R^6$, $R^7$, $R^9$ und $R^{10}$ | gleich oder verschieden sind und Wasserstoff, Methyl oder Alkyl mit 1-4 C-Atomen bedeuten, |
| $R^8$ und $R^{8'}$ | gleich oder verschieden sind und Alkyl mit 1-4 C-Atomen bedeuten, und |
| a und a' | gleich oder verschieden sind und eine Zahl 2 oder 3 bedeuten, |
| $R^2$ | für Nitro, Cyano oder Formyl steht, |

oder

| | |
|---|---|
| $R^1$ und $R^2$ | gemeinsam einen Lactonring der Formel 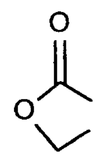 bilden, |
| $R^3$ | für Phenyl steht, das gegebenenfalls durch Fluor, Chlor, Nitro, Trifluormethyl, Alkyl oder Alkoxy mit bis zu 4 C-Atomen substituiert ist, |
| $R^4$ | für eine Gruppe der Formel $-CO-NR^{12}R^{13}$ oder $-CO-A-R^{14}$ steht, worin |
| $R^{12}$ und $R^{13}$ | gleich oder verschieden sind und Wasserstoff oder Phenyl oder einen geradkettigen, verzweigten, cyclischen, gesättigten oder ungesättigten Kohlenwasserstoffrest mit bis zu 4 Kohlenstoffatomen bedeuten, der gegebenenfalls durch Phenyl substituiert ist, das seinerseits durch Fluor, Chlor, Alkyl oder Alkoxy mit bis zu 4 C-Atomen substituiert sein kann, |
| A | eine direkte Bindung oder ein Sauerstoffatom bedeutet, |
| $R^{14}$ | Wasserstoff oder Phenyl bedeutet, oder einen cyclischen, geradkettigen oder verzweigten, gesättigten oder ungesättigten Kohlenwasserstoffrest mit bis zu 8 Kohlenstoffatomen bedeutet, der gegebenenfalls durch Sauerstoff oder Schwefel oder durch -CO-NH-, -O-CO-, -CO-O-, -NH-CO- oder $-NR^{18}$ unterbrochen ist, worin |
| $R^{18}$ | Wasserstoff, Phenyl oder geradkettiges oder verzweigtes Alkyl mit bis zu 4 Kohlenstoffatomen bedeutet, das gegebenenfalls durch Phenyl substituiert ist, und wobei der Kohlenwasserstoffrest gegebenenfalls durch Cyclopropyl, Cyclobutyl, Cyclopentyl, Cyclohexyl, Fluor, Chlor, Cyano, Hydroxy, Phenyl, Phenoxy, Phenylthio oder Pyridyl substituiert ist, oder durch eine Gruppe der Formel $-CO_2-R^{19}$, $-CONR^{20}R^{21}$ oder $-NR^{22}R^{23}$ substituiert ist, worin |
| $R^{19}$ | die oben angegebene Bedeutung von $R^{18}$ hat und mit dieser gleich oder verschieden ist und |
| $R^{20}$, $R^{21}$, $R^{22}$ und $R^{23}$ | die oben angegebene Bedeutung von $R^{12}$ und $R^{13}$ haben und mit diesen gleich oder verschieden sind, |

und deren Salze,
mit der Maßgabe, daß $R^5$ nicht für unsubstituiertes Alkyl stehen darf, wenn $R^1$ gleichzeitig unsubstituiertes Alkyl bedeutet oder $R^1$ und $R^2$ gemeinsam einen Lactonring bilden.

Ganz besonders bevorzugt sind Verbindungen der allgemeinen Formel (I),
in welcher

$R^1$ und $R^5$ gleich oder verschieden sind und für Wasserstoff, Formyl, Cyano, Trifluormethyl oder Methyl stehen, das gegebenenfalls durch Hydroxy, Methoxy, Acetoxy, Amino oder durch einen Rest der Formel $O-(CH_2)_a-OCH_3$ oder $-O-(CH_2)_{a'}-NH_2$ substituiert ist,
worin

a und a' gleich oder verschieden sind und eine Zahl 2 oder 3 bedeuten,

$R^2$ für Nitro oder Cyano steht,

oder

$R^1$ und $R^2$ gemeinsam für einen Lactonring der Formel

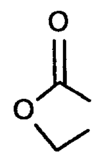

stehen,

$R^3$ für Phenyl steht, das gegebenenfalls durch Fluor, Chlor, Nitro, Trifluormethyl, Methyl oder Methoxy substituiert ist,

$R^4$ für eine Gruppe der Formel $-CO-A-R^{14}$ steht,
worin

A eine direkte Bindung oder ein Sauerstoffatom bedeutet
und

$R^{14}$ einen geradkettigen oder verzweigten, gesättigten Kohlenwasserstoffrest mit bis zu 6 Kohlenstoffatomen bedeutet, der gegebenenfalls durch Sauerstoff unterbrochen ist und der gegebenenfalls durch Cyclopropyl, Cyclobutyl, Cyclopentyl, Cyano, Phenyl, Pyridyl oder Phenoxy substituiert ist, oder
durch eine Gruppe $-NR^{22}R^{23}$ substituiert ist,
worin

$R^{22}$ und $R^{23}$ gleich oder verschieden sind und Wasserstoff, geradkettiges oder verzweigtes Alkyl mit bis zu 4 Kohlenstoffatomen, Phenyl oder Benzyl bedeuten

und deren Salze,
mit der Maßgabe, daß $R^5$ nicht für unsubstituiertes Alkyl stehen darf, wenn $R^1$ gleichzeitig unsubstituiertes Alkyl bedeutet oder $R^1$ und $R^2$ gemeinsam einen Lactonring bilden.

Die Erfindung betrifft auch Verfahren zur Herstellung der erfindungsgemäßen Verbindungen der allgemeinen Formel (I), dadurch gekennzeichnet,
daß man im Fall, daß $R^1$ und $R^2$ die oben angegebene Bedeutung haben, aber nicht gemeinsam einen Lactonring bilden,

[A] Verbindungen der allgemeinen Formel (II)

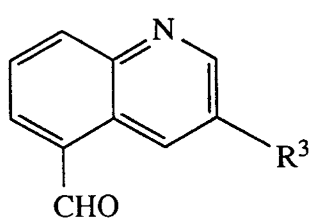

(II)

in welcher

$R^3$ die oben angegebene Bedeutung hat,

zunächst mit Acylderivaten der allgemeinen Formel (III)

$R^5-CO-CH_2-R^4$ (III)

in welcher

$R^4$ und $R^5$ die oben angegebene Bedeutung haben,

gegebenenfalls unter Isolierung der entsprechenden Ylidenverbindungen der allgemeinen Formel (IV)

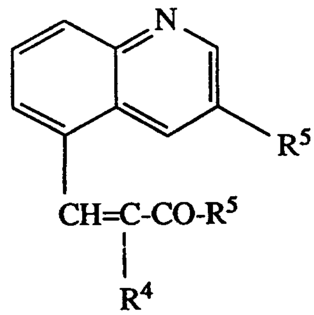
(IV)

in welcher

$R^3$, $R^4$ und $R^5$ die oben angegebene Bedeutung haben,

umsetzt und anschließend entweder mit Verbindungen der Formel (V)

$R^1$-CO-$CH_2$-$R^2$ (V)

in welcher

$R^1$ und $R^2$ die oben angegebene Bedeutung haben,

in Anwesenheit von Ammoniak oder Ammoniumsalzen, oder direkt mit Aminoderivaten der allgemeinen Formel (VI)

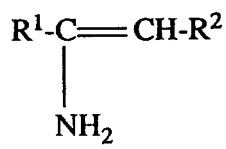
(VI)

in welcher

$R^1$ und $R^2$ die oben angegebene Bedeutung haben,

gegebenenfalls in Gegenwart von inerten organischen Lösemitteln umsetzt,

oder

[B] die Aldehyde der allgemeinen Formel (II) zunächst mit den Verbindungen der allgemeinen Formel (V), gegebenenfalls unter Isolierung der Ylidenverbindungen der allgemeinen Formel (VII)

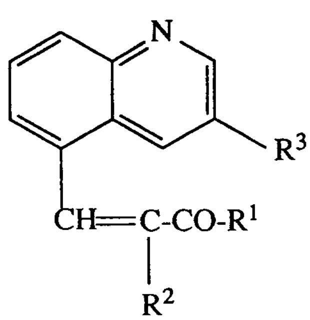
(VII)

in welcher

$R^1$, $R^2$ und $R^3$ die oben angegebene Bedeutung haben,

umsetzt und in einem nächsten Schritt mit den oben aufgeführten Verbindungen der allgemeinen Formel (III) in inerten Lösemitteln, in Anwesenheit von Ammoniak oder Ammoniumsalzen oder direkt mit Enaminocarbonsäurederivaten der allgemeinen Formel (VIII)

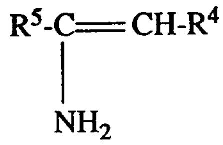

(VIII)

in welcher
$R^4$ und $R^5$ die oben angegebene Bedeutung haben,
umsetzt,
oder im Fall, daß $R^1$ und $R^2$ gemeinsam einen Lactonring bilden,
[C] zunächst nach denen unter [A] und [B] aufgeführten Methoden, Verbindungen der allgemeinen Formel (IX)

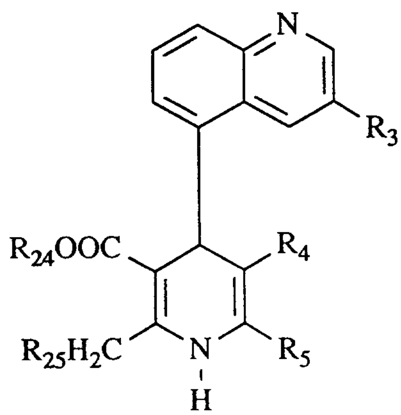

(IX)

in welcher
$R^3$, $R^4$ und $R^5$ die oben angegebene Bedeutung haben,
$R^{24}$ für einen $C_1$-$C_6$-Alkyl-Rest steht
und
$R^{25}$ für eine übliche Abgangsgruppe wie beispielsweise Chlor oder Acetoxy steht,
herstellt und nach üblichen Verfahren einen säure- oder basenkatalysierten Ringschluß anschließt.

Die erfindungsgemäßen Verfahren können durch folgendes Formelschema erläutert werden:

[A]

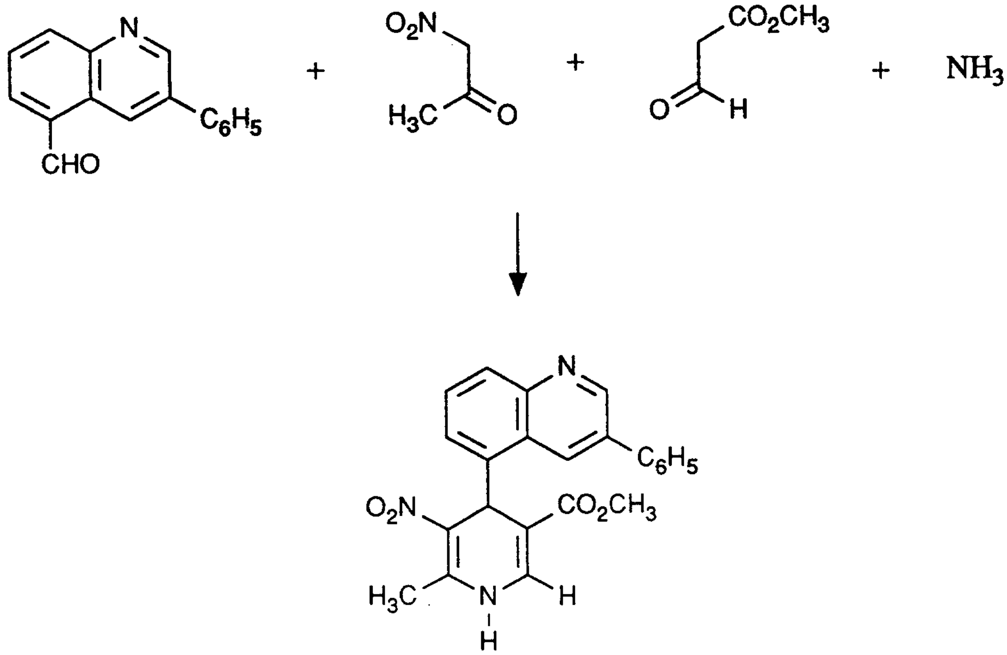

[B]

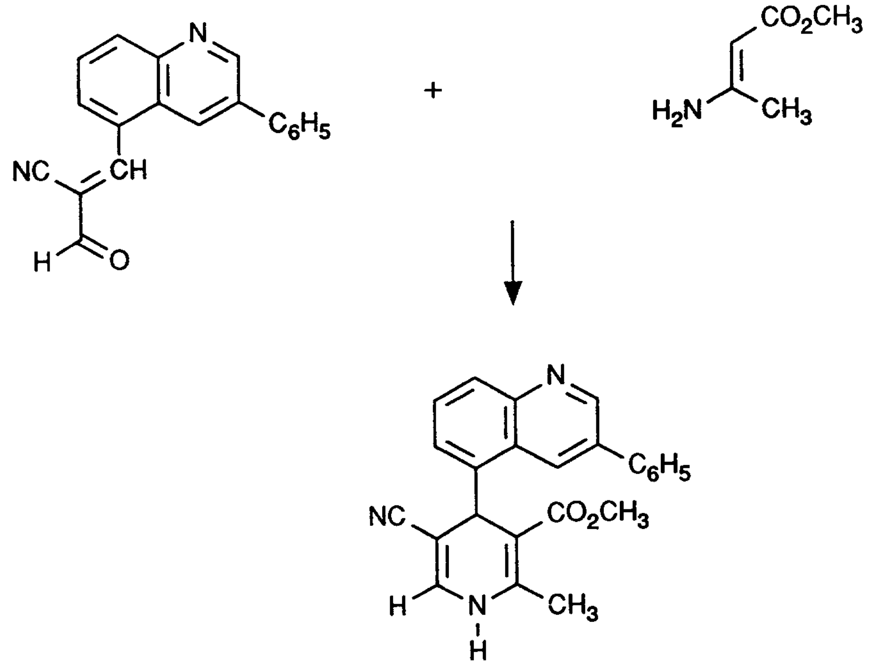

[C]

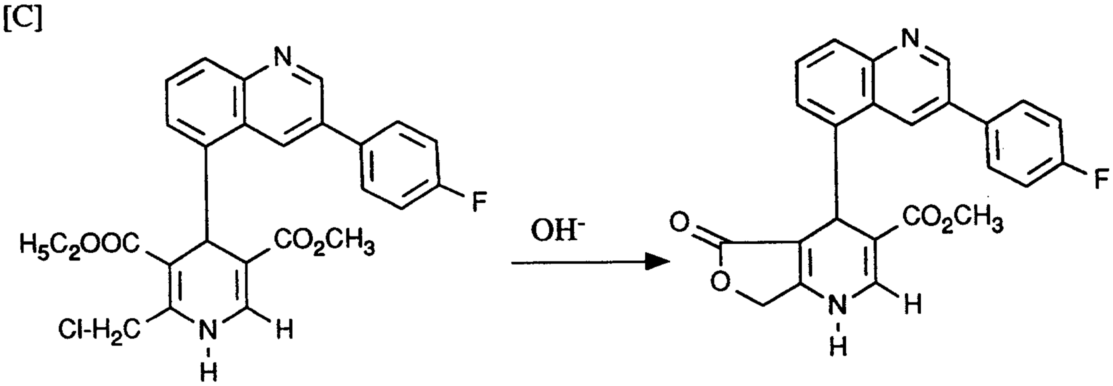


Als Lösemittel eignen sich für die Verfahren [A], [B] und [C] alle inerten organischen Lösemittel. Hierzu gehören bevorzugt Alkohole wie Methanol, Ethanol, n-bzw. iso-Propanol, Ether wie Diethylether, Tetrahydrofuran, Dioxan oder Glykolmono- oder Dimethylether, Eisessig, Pyridin, Dimethylformamid, Dimethylsulfoxid, Acetonitril oder Hexamethylphosphorsäuretriamid, Toluol oder Essigsäurealkylester.

Die Reaktionstemperatur für die Verfahren [A], [B] und [C] können in einem größeren Bereich variiert werden. Im allgemeinen arbeitet man in einem Bereich von 10 °C bis 200 °C, bevorzugt von 20 °C bis 150 °C.

Die Verfahren können bei Normaldruck, erhöhtem oder erniedrigtem Druck (beispielsweise von 0,5 bis 5 bar), vorzugsweise bei Normaldruck durchgeführt werden.

Bei der Durchführung der erfindungsgemäßen Verfahren ist das Verhältnis der an der Reaktion beteiligten Stoffe beliebig. Im allgemeinen arbeitet man jedoch bei molaren Mengen der Reaktanden.

Enantiomerenreine Formen erhält man z.B., indem man Diastereomerengemische der Verbindungen der allgemeinen Formel (I), in welcher $R^4$ für einen optischen Esterrest steht, nach üblicher Methode trennt, anschließend die enantiomerenreinen Carbonsäuren herstellt und dann beispielsweise durch Veresterung mit entsprechenden Alkoholen in die enantiomerenreinen Dihydropyridincarbonsäureester überführt.

Geeignet als chirale Esterreste sind alle Ester enantiomerenreiner Alkohole wie beispielsweise 2-Butanol, 1-Phenylethanol, Milchsäure, Milchsäureester, Mandelsäure, Mandelsäureester, 2-Aminoalkohole, Zuckerderivate und viele andere enantiomerenreine Alkohole mehr.

Die Trennung der Diastereomeren erfolgt im allgemeinen entweder durch fraktionierte Kristallisation, durch Säulenchromatographie oder durch Craig-Verteilung. Welches das optimale Verfahren ist, muß von

Fall zu Fall entschieden werden, manchmal ist es auch zweckmäßig, Kombinationen der einzelnen Verfahren zu benutzen. Besonders geeignet ist die Trennung durch Kristallisation oder Craig-Verteilung bzw. eine Kombination beider Verfahren.

Die Aldehyde der allgemeinen Formel (II) sind zum Teil bekannt [DOS 4 011 105] oder können nach üblichen Methoden hergestellt werden.

Die Verbindungen der Formeln (III), (IV), (V), (VI), (VII), (VIII) and (IX) sind bekannt oder können ebenfalls nach üblichen Methoden hergestellt werden.

Die vorstehenden Herstellungsverfahren sind lediglich zur Verdeutlichung angegeben. Die Herstellung der Verbindungen der Formel (I) sind nicht auf diese Verfahren beschränkt, sondern jede Modifikation dieser Verfahren ist in gleicher Weise für die Herstellung der erfindungsgemäßen Verbindungen anwendbar.

Die erfindungsgemäßen Verbindungen zeigen ein nicht vorhersehbares, wertvolles pharmakologisches Wirkspektrum. Sie beeinflussen die Kontraktionskraft des Herzens und den Tonus der glatten Muskulatur.
Sie können deshalb in Arzneimitteln zur Beeinflussung des pathologisch veränderten Blutdrucks, als Koronartherapeutika und zur Behandlung der Herzinsuffizienz eingesetzt werden. Darüber hinaus können sie zur Behandlung von Herzrhythmusstörungen, zur Senkung des Blutzuckers, zur Abschwellung von Schleimhäuten und zur Beeinflussung des Salz- und Flüssigkeitshaushaltes verwendet werden.

Die Herz- und Gefäßwirkungen wurden am isoliert perfundierten Herzen des Meerschweinchens gefunden. Dazu werden die Herzen von 250 bis 350 g schweren Meerschweinchen verwendet. Die Tiere werden mit einem Schlag auf den Kopf getötet, der Thorax geöffnet, und in die freipräparierte Aorta eine Metallkanüle eingebunden. Das Herz wird mit den Lungen aus dem Thorax herausgetrennt und über eine Aortenkanüle an die Perfusionsapparatur bei laufender Perfusion angeschlossen. Die Lungen werden an den Lungenwurzeln abgetrennt. als Perfusionsmedium dient eine Krebs-Henseleit-Lösung (1) (118,5 mmol/l NaCl, 4,75 mmol/l KCl, 1,19 mmol/l $KH_2PO_4$, 1,19 mmol/l $MgSO_4$, 25 mmol/l $NaHCO_3$, 0,013 mmol/l $Na_2EDTA$), deren $CaCl_2$-Gehalt 1,2 mmol/l beträgt. Als energielieferndes Substrat werden 10 mmol/l Glucose zugesetzt. vor der Perfusion wird die Lösung partikelfrei filtriert. Die Lösung wird mit Carbogen (95% $O_2$, 5% $CO_2$) zur Aufrechterhaltung des pH-Wertes 7,4 begast. Die Herzen werden mit konstantem Fluß (10 ml/min) bei 32 °C mittels einer Rollenquetschpumpe perfundiert.

Zur Messung der Herzfunktion wird ein flüssigkeitsgefüllter Latexballon, der über eine Flüssigkeitssäule mit einem Druckaufnehmer verbunden ist, durch den linken Vorhof in den linken Ventrikel eingeführt, und die isovolumetrischen Kontraktionen auf einem Schnellschreiber registriert. Der Perfusionsdruck wird mittels eines Druckaufnehmers, der vor dem Herzen mit dem Perfusionssystem in Verbindung steht, registiert. Unter diesen Bedingungen zeigt eine Senkung des Perfusionsdrucks eine Koronardilatation, eine Zu- bzw. Abnahme der linksventrikulären Kontraktionsamplitude eine Senkung bzw. einen Anstieg der Herzkontraktilität an. Die erfindungsgemäßen Verbindungen werden in geeigneten Verdünnungen in das Perfusionssystem kurz vor dem isolierten Herzen perfundiert.

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbei soll die therapeutisch wirksame Verbindung jeweils in einer Konzentration von etwa 0,5 bis 90-Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Die Applikation erfolgt in üblicher Weise, vorzugsweise oral oder parenteral, insbesondere perlingual oder intravenös.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Appli kation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament , der Art von dessen Formulierung und dem Zeitpunkt bzw. Intervall, zu welchen die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

Beispiel 1

3-Cyano-6-methyl-4-(3-phenylchinolin-5-yl)-1,4-dihydropyridin-5-carbonsäurepropylester

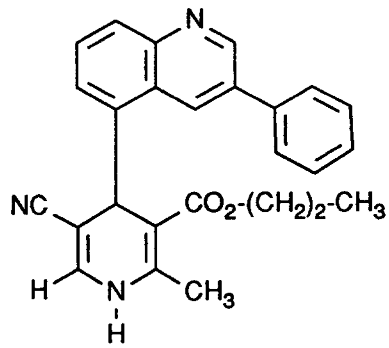

2,33 g (10 mmol) 3-Phenylchinolin-5-carbaldehyd werden in 20 ml n-Propanol mit 0,69 g (10 mmol) Isoxazol versetzt. In diese Suspension wird eine Lösung von 0,23 g (10 mmol) Natrium in 6 ml n-Propanol getropft. Es wird 4 Stunden bei 40 °C gerührt, mit 1,43 g (10 mmol) 3-Aminocrotonsäurepropylester und 0,6 ml (10 mmol) Essigsäure versetzt und 24 h zum Rückfluß erhitzt. Es wird abgekühlt, eingeengt und in Essigester/Wasser aufgenommen. Es wird abgetrennt, die Essigesterphase wird mit Wasser gewaschen, getrocknet und eingeengt. Das erhaltene Rohprodukt wird durch Flash-Chromatographie mit Toluol/Essigester-Gemischen gereinigt. Nach Kristallisation mit Acetonitril erhält man 1,4 g farblose Kristalle vom Schmelzpunkt 217 - 218 °C.

Beispiel 2 (Zwischenprodukt für Beispiel 3)

3-Acetyl-6-acetoxymethyl-4-(3-phenylchinolin-5-yl)-1,4-dihydropyridin-5-carbonsäureethylester

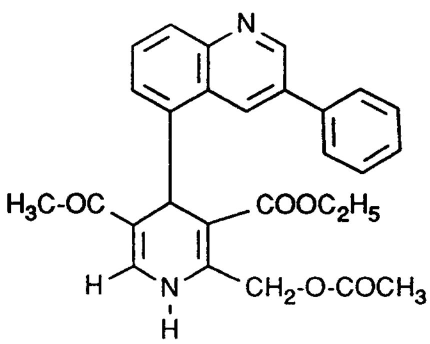

2,017 g (5 mmol) 2-(3-Phenylchinol-5-yliden)-4-acetoxy-3-oxo-buttersäureethylester werden mit 0,47 g (5,5 mmol) 4-Amino-3-buten-2-on und 0,33 ml Essigsäure in 40 ml Isopropanol 24 Stunden gekocht. Es wird abgekühlt, die ausgefallenen Kristalle werden abgesaugt und mit Isopropanol gewaschen. Man erhält 0,76 g des Zwischenproduktes vom Schmelzpunkt 234-235 °C.

Beispiel 3

3-Acetyl-4-(3-phenylchinolin-5-yl)-5-oxo-1,4,5,7-tetrahydrofuro[3,4-b]pyridin

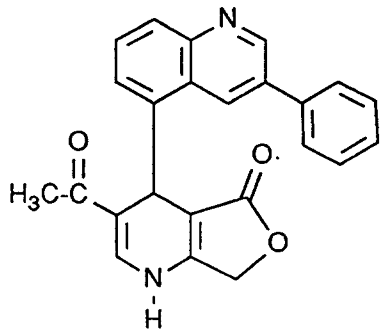

476 mg der Verbindung aus Beispiel 2 werden in 12 ml Methanol mit 9,25 mg Kaliumhydroxid versetzt und 2 Stunden zum Sieden erhitzt. Es wird abgekühlt und durch tropfenweise Zugabe von 1 N Salzsäure neutral gestellt. Das kristalline Produkt wird abgesaugt und mit Methanol/Wasser gewaschen. Man erhält 310 mg farblose Kristalle vom Schmelzpunkt 204-205 ° C.

In Analogie zu den Vorschriften der Beispiele 1, 2 und 3 werden die in Tabelle 1 aufgeführten Beispiele hergestellt:

Tabelle 1:

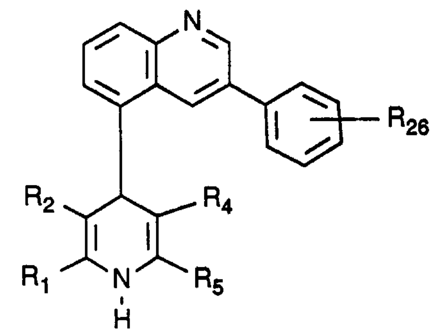

| Bsp.-Nr. | $R^1$ | $R^2$ | $R^4$ | $R^5$ | $R^{26}$ | F°C |
|---|---|---|---|---|---|---|
| 4 | $-CH_2O-CH_3$ | $-NO_2$ | $-CO_2CH_3$ | $-CH_3$ | H | 115 |
| 5 | $-CH_2-O-(CH_2)_2-OCH_3$ | $-NO_2$ | $-CO_2CH_3$ | $-CH_3$ | H | |
| 6 | $-CH_3$ | -CN | $-CO_2C_2H_5$ | $-CH_2-O-CO-CH_3$ | H | 186 |
| 7 | $-CH_3$ | -CN | $-CO_2C_2H_5$ | $-CH_2-O-CO-CH_3$ | Cl | 120 |
| 8 | $-CH_3$ | -CN | $-COCH_3$ | H | F | 262 |

<u>Fortsetzung Tabelle 1:</u>

| Bsp.-Nr. | $R^1$ | $R^2$ | $R^4$ | $R^5$ | $R^{26}$ | F°C | Enantiomer |
|---|---|---|---|---|---|---|---|
| 9 | $-CH_3$ | -CN | $-CO-CH_3$ | H | H | 278 | |
| 10 | $-CH_3$ | -CN | $-CO_2-CH_3$ | H | H | | |
| 11 | H | -CN | $-CO_2-(CH_2)_2-CH_3$ | $-CH_3$ | H | 171-173 | (-) |
| 12 | H | -CN | $-CO_2-(CH_2)_2-CH_3$ | $-CH_3$ | H | 171-174 | (+) |
| 13 | $-CH_3$ | -CN | $-CO_2CH_3$ | $-CF_3$ | H | 139 | |
| 14 | $-CH_3$ | -CN | $-CO_2CH(CH_3)_2$ | $-CF_3$ | H | 212 | |
| 15 | $-CH_3$ | -CN | $-CO_2C_2H_5$ | $-CF_3$ | H | 182 | |
| 16 | $-CH_3$ | -CN | $-CO_2C_2H_5$ | $-CH_2NH_2$ | H | | |
| 17 | $-CH_3$ | -CN | $-CO_2C_2H_5$ | $-CH_2-O(CH_2)_2-NH_2$ | H | 162 | |
| 18 | $-CH_3$ | -CN | $-CO_2-CH(CH_3)_2$ | $-CH_2-O-(CH_2)_3-NH_2$ | H | Schaum | |

**Patentansprüche**

**1.** 2,6-Disubstituierte 4-Chinolyldihydropyridine der allg. Formel (I)

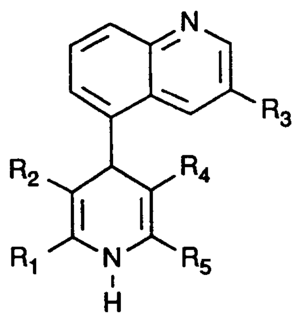

(I)

in welcher

$R^1$ und $R^5$ gleich oder verschieden sind und für Wasserstoff, Cyano, Formyl, Trifluormethyl oder für geradkettiges oder verzweigtes Alkyl mit bis zu 8 Kohlenstoffatomen stehen, das gegebenenfalls durch Hydroxy, geradkettiges oder verzweigtes Alkoxy oder Alkoxycarbonyl mit jeweils bis zu 8 Kohlenstoffatomen oder durch eine Gruppe der Formel $-NR^6R^7$, $-O-CO-R^8$, $-O-(CH_2)_a-OR^{8'}$ oder $-O-(CH_2)_{a'}-NR^9R^{10}$ substituiert ist, worin

$R^6$, $R^7$, $R^9$ und $R^{10}$ gleich oder verschieden sind und Wasserstoff, Phenyl oder geradkettiges oder verzweigtes Alkyl mit bis zu 6 Kohlenstoffatomen bedeuten,

$R^8$ und $R^{8'}$ gleich oder verschieden sind und geradkettiges oder ver zweigtes Alkyl mit bis zu 6 Kohlenstoffatomen bedeuten,

und

a und a' gleich oder verschieden sind und eine Zahl 2, 3, 4 oder 5 bedeuten,

$R^2$ für Nitro, Cyano oder Formyl steht,

oder

$R^1$ und $R^2$ gemeinsam einen Lactonring der Formel

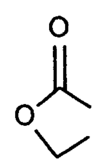

bilden,

$R^3$ für Aryl mit 6 bis 10 Kohlenstoffatomen steht, das gegebenenfalls bis zu 3-fach gleich oder verschieden durch Halogen, Nitro, Cyano, Hydroxy, Amino, Trifluormethyl, Trifluormethoxy, Trifluormethylthio, geradkettiges oder verzweigtes Alkyl, Alkoxy oder Alkoxycarbonyl mit jeweils bis zu 8 Kohlenstoffatomen oder Carboxy substituiert ist, oder für Thienyl oder Pyridyl steht, die gegebenenfalls durch Halogen substituiert sind,

$R^4$ für eine Gruppe der Formel $-CO-NR^{12}R^{13}$, $-CO-A-R^{14}$ oder $-P(O)(OR^{15})-(OR^{16})$ steht, worin

$R^{12}$ und $R^{13}$ gleich oder verschieden sind und Wasserstoff oder einen geradkettigen, verzweigten, cyclischen, gesättigten oder ungesättigten Kohlenwasserstoffrest mit bis zu 10 Kohlenstoffatomen bedeuten, der gegebenenfalls durch Cycloalkyl mit 3 bis 8 Kohlenstoffatomen, Halogen, Hydroxy, Cyano oder durch Aryl, Aryloxy, Arylthio mit jeweils 6 bis 10 Kohlenstoffatomen oder durch einen 5- bis 7-gliedrigen, gesättigen oder ungesättigten Heterocyclus mit bis zu 3 Heteroatomen aus der Reihe S, N oder O substituiert ist, wobei die Cyclen ihrerseits durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert sein können, oder Aryl mit 6 bis 10 Kohlenstoffatomen oder einen 5- bis 7-gliedrigen, gesättigten oder ungesättigten Heterocyclus mit bis zu 3 Heteroatomen aus der Reihe S, N oder O bedeuten, die gegebenenfalls bis zu 2-fach gleich oder verschieden durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert sind,

oder

$R^{12}$ und $R^{13}$ gemeinsam unter Einbezug des Stickstoffatoms einen 5- bis 8-gliedrigen, gesättigen oder ungesättigten Heterocyclus bilden, der gegebenenfalls durch Sauerstoff oder durch Formel $S(O)_b$, -CO- oder $-NR^{17}$ unterbrochen ist,

worin

b eine Zahl 0, 1 oder 2 bedeutet,

$R^{17}$ Wasserstoff oder Aryl mit 6 bis 10 Kohlenstoffatomen bedeutet, das gegebenenfalls bis zu 2-fach gleich oder verschieden durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert ist, oder

einen cyclischen, geradkettigen oder verzweigten, gesättigten oder ungesättigten Kohlenwasserstoffrest mit bis zu 8 Kohlenstoffatomen bedeutet, der gegebenenfalls durch Hydroxy, Halogen oder durch Aryl mit 6 bis 10 Kohlenstoffatomen oder einen 5- bis 7-gliedrigen, gesättigten oder ungesättigten Heterocyclus mit 3 Heteroatomen aus der Reihe S, N oder O substituiert ist, die ihrerseits bis zu 2-fach gleich oder verschieden durch Halogen, Cyano oder durch geradkettiges oder verzweigtes Alkyl,, Alkoxy, Alkylthio, Alkoxycarbonyl, Halogenalkyl, Halogenalkoxy oder Halogenalkylthio mit jeweils bis zu 4 Kohlenstoffatomen substituiert sein können,

und der Heterocyclus gegebenenfalls durch geradkettiges oder verzweigtes Alkoxy oder Alkylthio mit jeweils bis zu 4 Kohlenstoffatomen, Halogen, Ary, lmit 6 bis 10 Kohlenstoffatomen, einen 4- bis 7-gliedrigen, gesättigten oder ungesättigten Heterocyclus mit bis zu 3 Heteroatomen aus der Reihe S, N oder O oder durch geradkettiges oder verzweigtes Alkyl mit bis zu 4 Kohlenstoffatomen substituiert ist, das gegebenenfalls seinerseits durch Aryl mit 6 bis 10 Kohlenstoffatomen substituiert sein kann,

A eine direkte Bindung oder ein Sauerstoffatom bedeutet,

$R^{14}$ Wasserstoff oder Aryl mit 6 bis 10 Kohlenstoffatomen oder einen 4- bis 7-gliedrigen, gesättigten oder ungesättigten Heterocyclus mit bis zu 3 Heteroatomen aus der Reihe S, N oder O bedeutet, die gegebenenfalls bis zu 3-fach gleich oder verschieden durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert sind, oder

einen cyclischen, geradkettigen oder verzweigten, gesättigten oder ungesättigten Kohlenwasserstoffrest mit bis zu 12 Kohlenstoffatomen bedeutet, der gegebenenfalls bis zu 3-fach gleich oder verschieden durch Sauerstoff -CO-, -CO-NH-, -O-CO-, -CO-O-, -NH-CO-, $-SO_2-NH-$, $-NH-SO_2-$, $-S(O)_c-$ oder $-NR^{18}$ unterbrochen ist,

worin

c die oben angegebene Bedeutung von b hat und mit dieser gleich oder verschieden ist,

$R^{18}$ die oben angegebene Bedeutung von $R^{17}$ hat und mit dieser gleich oder verschieden ist,

oder der bis zu 3-fach gleich oder verschieden unterbrochen ist durch Aryliden mit 6 bis 10 Kohlenstoffatomen oder einen cyclischen Rest der Formel

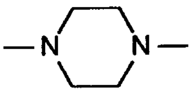 , 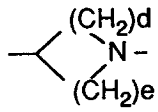 , 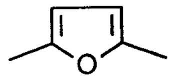 oder 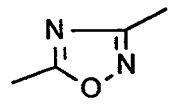

worin

d und e gleich oder verschieden sind und eine Zahl 1 oder 2 bedeuten,

und worin Aryliden und die Heterocyclen ihrerseits durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert sein können,

und wobei der Kohlenwasserstoffrest gegebenenfalls bis zu 3-fach gleich oder verschieden durch Cycloalkyl mit 3 bis 8 Kohlenstoffatomen, Halogen, Nitro, Cyano, Hydroxy, $-O-NO_2$, geradkettiges oder verzweigtes Alkylthio, Alkoxy oder Acyloxy mit jeweils bis zu 8 Kohlenstoffatomen oder durch Aryl, Aryloxy oder Arylthio mit jeweils 6 bis 10 Kohlenstoffatomen oder durch einen 5- bis 7-gliedrigen, gesättigten oder ungesättigen Heterocyclus mit bis zu 3 Heteroatomen aus der Reihe S, N oder O substituiert ist, wobei die Cyclen ihrerseits bis zu 3-fach gleich oder verschieden durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert sein können, oder der Kohlenwasserstoffrest durch eine Gruppe der Formel $-CO_2-R^{19}$, $-CONR^{20}R^{21}$ oder $-NR^{22}R^{23}$ substituiert ist,

worin

$R^{19}$ die oben angegebene Bedeutung von $R^{17}$ hat und mit dieser gleich oder verschieden ist

und

$R^{20}$, $R^{21}$, $R^{22}$ und $R^{23}$ die oben angegebene Bedeutung von $R^{12}$ und $R^{13}$ haben und mit diesen gleich oder verschieden sind,

$R^{15}$ und $R^{16}$ gleich oder verschieden sind und geradkettiges oder verzweigtes Alkyl mit bis zu 8 Kohlenstoffatomen bedeuten,

oder

$R^{15}$ und $R^{16}$ gemeinsam unter Einbezug der Sauerstoffatome und des Phosphors einen 5- bis 7-gliedrigen, gesättigen Heterocyclus bilden,

und deren Salze,

mit der Maßgabe, daß $R^5$ nicht für unsubstituiertes Alkyl stehen darf, wenn $R^1$ gleichzeitig unsubstituiertes Alkyl bedeutet oder $R^1$ und $R^2$ gemeinsam einen Lactonring bilden.

**2.** Verbindungen der allg. Formel (I) gem. Anspruch 1 in welcher

$R^1$ und $R^5$ gleich oder verschieden sind und für Wasserstoff, Cyano, Formyl, Trifluormethyl oder für geradkettiges oder verzweigtes Alkyl mit bis zu 6 Kohlenstoffatomen stehen, das gegebenenfalls durch Hydroxy, geradkettiges oder verzweigtes Alkoxy oder Alkoxycarbonyl mit jeweils bis zu 4 Kohlenstoffatomen oder durch eine Gruppe der Formel $-NR^6R^7$, $-O-CO-R^8$, $-O-(CH_2)_a-OR^{8'}$ oder $-O-(CH_2)_{a'}-NR^9R^{10}$ substituiert ist,

worin

$R^6$, $R^7$, $R^9$ und $R^{10}$ gleich oder verschieden sind und Wasserstoff, Phenyl oder geradkettiges oder verzweigtes Alkyl mit bis zu 4 Kohlenstoffatomen bedeuten,

$R^8$ und $R^{8'}$ gleich oder verschieden sind und geradkettiges oder verzweigtes Alkyl mit bis zu 4 Kohlenstoffatomen bedeuten,

und

a und a' gleich oder verschieden sind und eine Zahl 2, 3 oder 4 bedeuten,

$R^2$ für Nitro, Cyano oder Formyl steht,

oder

$R^1$ und $R^2$ gemeinsam einen Lactonring der Formel

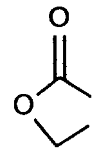

bilden,

$R^3$ für Phenyl steht, das gegebenenfalls durch Fluor, Chlor, Nitro, Cyano, Hydroxy, Amino, Trifluormethyl oder durch geradkettiges oder verzweigtes Alkyl oder Alkoxy mit jeweils bis zu 4 Kohlenstoffatomen substituiert ist, oder
für Thienyl oder Pyridyl steht, die gegebenenfalls durch Fluor, Chlor oder Brom substituiert sind,

$R^4$ für eine Gruppe der Formel $-CO-NR^{12}R^{13}$, $-CO-A-R^{14}$ oder $-P(O)(OR^{15})(OR^{16})$ steht,
worin

$R^{12}$ und $R^{13}$ gleich oder verschieden sind und Wasserstoff oder einen geradkettigen, verzweigten, cyclischen, gesättigten oder ungesättigten Kohlenwasserstoffrest mit bis zu 6 Kohlenstoffatomen bedeuten, der gegebenenfalls durch Cyclopropyl, Cyclopentyl, Cyclohexyl, Fluor, Chlor, Brom, Hydroxy oder durch Phenyl oder Pyridyl substituiert ist, wobei die Cyclen ihrerseits durch Fluor, Chlor, Brom, Cyano oder durch geradkettiges oder verzweigtes Alkyl, Alkoxy, Alkylthio, Alkoxycarbonyl mit jeweils bis zu 3 Kohlenstoffatomen, Trifluormethyl oder Trifluormethoxy substituiert sein können, oder Phenyl oder Pyridyl bedeuten, die gegebenenfalls durch Fluor, Chlor oder durch geradkettiges oder verzweigtes Alkyl, Alkoxy, Alkylthio, Alkoxycarbonyl mit jeweils bis zu 4 Kohlenstoffatomen, Trifluormethyl oder Trifluormethoxy substituiert sind,
oder

$R^{12}$ und $R^{13}$ gemeinsam unter Einbezug des Stickstoffatoms einen 5- bis 7-gliedrigen, gesättigten oder ungesättigten Heterocyclus bilden, der gegebenenfalls durch Sauerstoff oder durch $S(O)_b$,-CO- oder $-NR^{17}$ unterbrochen sein kann,
worin

b eine Zahl 0, 1 oder 2 bedeutet,

$R^{17}$ Wasserstoff oder Phenyl bedeutet, oder
einen cyclischen, geradkettigen oder verzweigten, gesättigten oder ungesättigten Kohlenwasserstoffrest mit bis zu 6 Kohlenstoffatomen bedeutet, der gegebenenfalls durch Fluor, Chlor, Brom oder durch Phenyl oder Pyridyl substituiert ist, die ihrerseits durch Fluor, Chlor, Methyl, Ethyl, Methoxy, Ethoxy, Trifluormethyl oder Trifluormethoxy substituiert sein können,

und der Heterocyclus gegebenenfalls durch geradkettiges oder verzweigtes Alkxoy oder Alkylthio mit jeweils bis zu 3 Kohlenstoffatomen, Fluor, Chlor, Brom, Phenyl oder Pyridyl oder durch verzweigtes Alkyl mit bis zu 4 Kohlenstoffatomen oder Benzyl substituiert ist,

A eine direkte Bindung oder ein Sauerstoffatom bedeutet,

$R^{14}$ Wasserstoff, Phenyl oder Pyridyl bedeutet, die gegebenenfalls durch Fluor, Chlor oder durch geradkettiges oder verzweigtes Alkyl, Alkoxy oder Alkylthio mit jeweils bis zu 3 Kohlenstoffatomen, Trifluormethyl oder Trifluormethoxy substituiert sind, oder
einen cyclischen, geradkettigen oder verzweigten, gesättigten oder ungesättigten Kohlenwasserstoffrest mit bis zu 10 Kohlenstoffatomen bedeutet, der gegebenenfalls bis zu 2-fach gleich oder verschieden durch Sauerstoff oder durch -CO-, -CO-NH-, -O-CO-, -CO-O-, -NH-CO-, $-SO_2-NH-$, $-NH-SO_2-$, $-S(O)_c$-oder $-NR^{18}$ unterbrochen ist,
worin

c die oben angegebene Bedeutung von b hat und mit dieser gleich oder verschieden ist,

$R^{18}$ die oben angegebene Bedeutung von $R^{17}$ hat und mit dieser gleich oder verschieden ist,

oder der bis zu 2-fach gleich oder verschieden unterbrochen ist durch Phenyliden oder einen cyclischen Rest der Formel

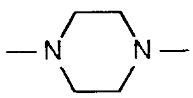 , 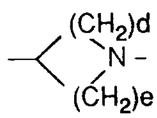 , 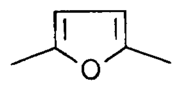 oder 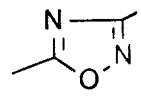

worin

d und e gleich oder verschieden sind und eine Zahl 1 oder 2 bedeuten,

und wobei der Kohlenwasserstoffrest gegebenenfalls bis zu 2-fach gleich oder verschieden durch Cyclopropyl, Cyclobutyl, Cyclopentyl, Cyclohexyl, Fluor, Chlor, Brom, Nitro, Cyano, Hydroxy, $-O-NO_2$, geradkettiges oder verzweigtes Alkylthio, Alkoxy oder Acyloxy mit jeweils bis zu 6 Kohlenstoffatomen oder durch Phenyl, Phenoxy, Phenylthio oder Pyridyl substituiert ist, wobei die Cyclen ihrerseits bis zu 2-fach gleich oder verschieden durch Fluor, Chlor, Brom, Cyano oder durch geradkettiges oder verzweigtes Alkyl, Alkoxy, Alkylthio, Alkoxycarbonyl mit jeweils mit bis zu 4 Kohlenstoffatomen, Trifluormethyl oder Trifluormethoxy substituiert sind, oder

der Kohlenwasserstoffrest durch eine Gruppe der Formel $-CO_2-R^{19}$, $-CONR^{20}R^{21}$ oder $-NR^{22}R^{23}$ substituiert ist,

worin

$R^{19}$ die oben angegebene Bedeutung von $R^{17}$ hat und mit dieser gleich oder verschieden ist

und

$R^{20}$, $R^{21}$, $R^{22}$ und $R^{23}$ die oben angegebene Bedeutung von $R^{12}$ und $R^{13}$ haben und mit diesen gleich oder verschieden sind,

$R^{15}$ und $R^{16}$ gleich oder verschieden sind und geradkettiges oder verzweigtes Alkyl mit bis zu 6 Kohlenstoffatomen bedeuten

oder

$R^{15}$ und $R^{16}$ gemeinsam unter Einbezug der Sauerstoffatome und des Phosphors einen 6-gliedrigen gesättigten Cyclus bilden

und deren Salze,

mit der Maßgabe, daß $R^5$ nicht für unsubstituiertes Alkyl stehen darf, wenn $R^1$ gleichzeitig unsubstituiertes Alkyl bedeutet oder $R^1$ und $R^2$ gemeinsam einen Lactonring bilden.

**3.** Verbindungen der allg. Formel (I) gem. Anspruch 1 in welcher

$R^1$ und $R^5$ gleich oder verschieden sind und für Wasserstoff, Cyano, Formyl, Trifluormethyl oder Methyl oder Ethyl stehen, die gegebenenfalls durch Hydroxy, geradkettiges oder verzweigtes Alkoxy oder Alkoxycarbonyl mit jeweils bis zu 4 Kohlenstoffatomen oder durch eine Gruppe der Formel $-NR^6R^7$, $-O-CO-R^8$, $-O-(CH_2)_a-OR^{8'}$ oder $-O-(CH_2)_{a'}-NR^9R^{10}$ substituiert sind,

worin

$R^6$, $R^7$, $R^9$ und $R^{10}$ gleich oder verschieden sind und Wasserstoff, Methyl oder Alkyl mit 1-4 C-Atomen bedeuten,

$R^8$ und $R^{8'}$ gleich oder verschieden sind und Alkyl mit 1-4 C-Atomen bedeuten,

und

a und a' gleich oder verschieden sind und eine Zahl 2 oder 3 bedeuten,

$R^2$ für Nitro, Cyano oder Formyl steht,

oder

$R^1$ und $R^2$ gemeinsam einen Lactonring der Formel

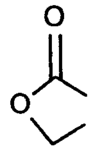

bilden,

$R^3$ für Phenyl steht, das gegebenenfalls durch Fluor, Chlor, Nitro, Trifluormethyl, Alkyl oder Alkoxy mit bis zu 4 C-Atomen substituiert ist,

$R^4$ für eine Gruppe der Formel $-CO-NR^{12}R^{13}$ oder $-CO-A-R^{14}$ steht,

worin

$R^{12}$ und $R^{13}$ gleich oder verschieden sind und Wasserstoff oder Phenyl oder einen geradkettigen, verzweigten, cyclischen, gesättigten oder ungesättigten Kohlenwasserstoffrest mit bis zu 4 Kohlenstoffatomen bedeuten, der gegebenenfalls durch Phenyl substituiert ist, das seinerseits durch Fluor, Chlor, Alkyl oder Alkoxy mit bis zu 4 C-Atomen substituiert sein kann,

A eine direkte Bindung oder ein Sauerstoffatom bedeutet,

$R^{14}$ Wasserstoff oder Phenyl bedeutet, oder

einen cyclischen, geradkettigen oder verzweigten, gesättigten oder ungesättigten Kohlenwasserstoffrest mit bis zu 8 Kohlenstoffatomen bedeutet, der gegebenenfalls durch Sauerstoff oder Schwefel oder durch -CO-NH-, -O-CO-, -CO-O-, -NH-CO- oder $-NR^{18}$ unterbrochen ist,

worin

$R^{18}$ Wasserstoff, Phenyl oder geradkettiges oder verzweigtes Alkyl mit bis zu 4 Kohlenstoffatomen bedeutet, das gegebenenfalls durch Phenyl substituiert ist,

und wobei der Kohlenwasserstoffrest gegebenenfalls durch Cyclopropyl, Cyclobutyl, Cyclopentyl, Cyclohexyl, Fluor, Chlor, Cyano, Hydroxy, Phenyl, Phenoxy, Phenylthio oder Pyridyl substituiert ist, oder

durch eine Gruppe der Formel $-CO_2-R^{19}$, $-CONR^{20}R^{21}$ oder $-NR^{22}R^{23}$ substituiert ist,

worin

$R^{19}$ die oben angegebene Bedeutung von $R^{18}$ hat und mit dieser gleich oder verschieden ist

und

$R^{20}$, $R^{21}$, $R^{22}$ und $R^{23}$ die oben angegebene Bedeutung von $R^{12}$ und $R^{13}$ haben und mit diesen gleich oder verschieden sind,

und deren Salze,

mit der Maßgabe, daß $R^5$ nicht für unsubstituiertes Alkyl stehen darf, wenn $R^1$ gleichzeitig unsubstituiertes Alkyl bedeutet oder $R^1$ und $R^2$ gemeinsam einen Lactonring bilden.

**4.** Verbindungen der allg. Formel (I) gem. Anspruch 1 in welcher

$R^1$ und $R^5$ gleich oder verschieden sind und für Wasserstoff, Formyl, Cyano, Trifluormethyl oder Methyl stehen, das gegebenenfalls durch Hydroxy, Methoxy, Acetoxy, Amino oder durch einen Rest der Formel $O-(CH_2)_a-OCH_3$ oder $-O-(CH_2)_{a'}-NH_2$ substituiert ist,

worin

a und a' gleich oder verschieden sind und eine Zahl 2 oder 3 bedeuten,

$R^2$ für Nitro oder Cyano steht,

oder

$R^1$ und $R^2$ gemeinsam für einen Lactonring der Formel

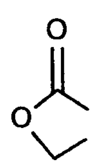

stehen,

$R^3$ für Phenyl steht, das gegebenenfalls durch Fluor, Chlor, Nitro, Trifluormethyl, Methyl oder Methoxy substituiert ist,

$R^4$ für eine Gruppe der Formel $-CO-A-R^{14}$ steht,

worin

A eine direkte Bindung oder ein Sauerstoffatom bedeutet

und

$R^{14}$ einen geradkettigen oder verzweigten, gesättigten Kohlenwasserstoffrest mit bis zu 6 Kohlenstoffatomen bedeutet, der gegebenenfalls durch Sauerstoff unterbrochen ist und der gegebenenfalls durch Cyclopropyl, Cyclobutyl, Cyclopentyl, Cyano, Phenyl, Pyridyl oder Phenoxy substituiert ist, oder durch eine Gruppe $-NR^{22}R^{23}$ substituiert ist,

worin

$R^{22}$ und $R^{23}$ gleich oder verschieden sind und Wasserstoff, geradkettiges oder verzweigtes Alkyl mit bis zu 4 Kohlenstoffatomen, Phenyl oder Benzyl bedeuten

und deren Salze,

mit der Maßgabe, daß $R^5$ nicht für unsubstituiertes Alkyl stehen darf, wenn $R^1$ gleichzeitig unsubstituiertes Alkyl bedeutet oder $R^1$ und $R^2$ gemeinsam einen Lactonring bilden.

**5.** Verfahren zur Herstellung von Verbindungen der allg. Formel (I)

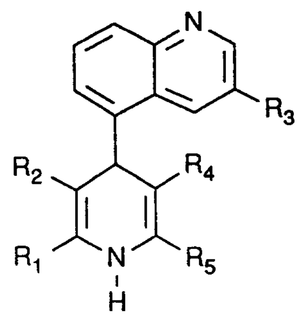

(I)

in welcher

$R^1$ und $R^5$ gleich oder verschieden sind und für Wasserstoff, Cyano, Formyl, Trifluormethyl oder für geradkettiges oder verzweigtes Alkyl mit bis zu 8 Kohlenstoffatomen stehen, das gegebenenfalls durch Hydroxy, geradkettiges oder verzweigtes Alkoxy oder Alkoxycarbonyl mit jeweils bis zu 8 Kohlenstoffatomen oder durch eine Gruppe der Formel $-NR^6R^7$, $-O-CO-R^8$, $-O-(CH_2)_a-OR^{8'}$ oder $-O-(CH_2)_{a'}-NR^9R^{10}$ substituiert ist,

worin

$R^6$, $R^7$, $R^9$ und $R^{10}$ gleich oder verschieden sind und Wasserstoff, Phenyl oder geradkettiges oder verzweigtes Alkyl mit bis zu 6 Kohlenstoffatomen bedeuten,

$R^8$ und $R^{8'}$ gleich oder verschieden sind und geradkettiges oder ver zweigtes Alkyl mit bis zu 6 Kohlenstoffatomen bedeuten,

und

a und a' gleich oder verschieden sind und eine Zahl 2, 3, 4 oder 5 bedeuten, $R^2$ für Nitro, Cyano oder Formyl steht,

oder

$R^1$ und $R^2$ gemeinsam einen Lactonring der Formel

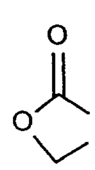

bilden,

$R^3$ für Aryl mit 6 bis 10 Kohlenstoffatomen steht, das gegebenenfalls bis zu 3-fach gleich oder verschieden durch Halogen, Nitro, Cyano, Hydroxy, Amino, Trifluormethyl, Trifluormethoxy, Trifluormethylthio, geradkettiges oder verzweigtes Alkyl, Alkoxy oder Alkoxycarbonyl mit jeweils bis zu 8 Kohlenstoffatomen oder Carboxy substituiert ist, oder

für Thienyl oder Pyridyl steht, die gegebenenfalls durch Halogen substituiert sind,

$R^4$ für eine Gruppe der Formel $-CO-NR^{12}R^{13}$, $-CO-A-R^{14}$ oder $-P(O)(OR^{15})(OR^{16})$ steht,

worin

$R^{12}$ und $R^{13}$ gleich oder verschieden sind und Wasserstoff oder einen geradkettigen, verzweigten, cyclischen, gesättigten oder ungesättigten Kohlenwasserstoffrest mit bis zu 10 Kohlenstoffatomen bedeuten, der gegebenenfalls durch Cycloalkyl mit 3 bis 8 Kohlenstoffatomen, Halogen, Hydroxy, Cyano oder durch Aryl, Aryloxy, Arylthio mit jeweils 6 bis 10 Kohlenstoffatomen oder durch einen 5- bis 7-gliedrigen, gesättigen oder ungesättigten Heterocyclus mit bis zu 3 Heteroatomen aus der Reihe S, N oder O substituiert ist, wobei die Cyclen ihrerseits durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert sein können, oder

Aryl mit 6 bis 10 Kohlenstoffatomen oder einen 5- bis 7-gliedrigen, gesättigten oder ungesättigten Heterocyclus mit bis zu 3 Heteroatomen aus der Reihe S, N oder O bedeuten, die gegebenenfalls bis zu 2-fach gleich oder verschieden durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert sind,

oder

$R^{12}$ und $R^{13}$ gemeinsam unter Einbezug des Stickstoffatoms einen 5- bis 8-gliedrigen, gesättigen oder ungesättigten Heterocyclus bilden, der gegebenenfalls durch Sauerstoff oder durch Formel $S(O)_b$, -CO- oder $-NR^{17}$ unterbrochen ist,

worin

b eine Zahl 0, 1 oder 2 bedeutet,

$R^{17}$ Wasserstoff oder Aryl mit 6 bis 10 Kohlenstoffatomen bedeutet, das gegebenenfalls bis zu 2-fach gleich oder verschieden durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert ist, oder

einen cyclischen, geradkettigen oder verzweigten, gesättigten oder ungesättigten Kohlenwasserstoffrest mit bis zu 8 Kohlenstoffatomen bedeutet, der gegebenenfalls durch Hydroxy, Halogen oder durch Aryl mit 6 bis 10 Kohlenstoffatomen oder einen 5- bis 7-gliedrigen, gesättigten oder ungesättigten Heterocyclus mit 3 Heteroatomen aus der Reihe S, N oder O substituiert ist, die ihrerseits bis zu 2-fach gleich oder verschieden durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert sein können,

und der Heterocyclus gegebenenfalls durch geradkettiges oder verzweigtes Alkoxy oder Alkylthio mit jeweils bis zu 4 Kohlenstoffatomen, Halogen, Ary lmit 6 bis 10 Kohlenstoffatomen, einen 4- bis 7-gliedrigen, gesättigten oder ungesättigten Heterocyclus mit bis zu 3 Heteroatomen aus der Reihe S, N oder O oder durch geradkettiges oder verzweigtes Alkyl mit bis zu 4 Kohlenstoffatomen substituiert ist, das gegebenenfalls seinerseits durch Aryl mit 6 bis 10 Kohlenstoffatomen substituiert sein kann,

A eine direkte Bindung oder ein Sauerstoffatom bedeutet,

$R^{14}$ Wasserstoff oder Aryl mit 6 bis 10 Kohlenstoffatomen oder einen 4- bis 7-gliedrigen, gesättigten oder ungesättigten Heterocyclus mit bis zu 3 Heteroatomen aus der Reihe S, N oder O bedeutet, die gegebenenfalls bis zu 3-fach gleich oder verschieden durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit

jeweils bis zu 4 Kohlenstoffatomen substituiert sind, oder
einen cyclischen, geradkettigen oder verzweigten, gesättigten oder ungesättigten Kohlenwasserstoffrest mit bis zu 12 Kohlenstoffatomen bedeutet, der gegebenenfalls bis zu 3-fach gleich oder verschieden durch Sauerstoff -CO-, -CO-NH-, -O-CO-, -CO-O-, -NH-CO-, $-SO_2$-NH-, $-NH-SO_2-$, $-S(O)_c$-oder $-NR^{18}$ unterbrochen ist,
worin

c die oben angegebene Bedeutung von b hat und mit dieser gleich oder verschieden ist,

$R^{18}$ die oben angegebene Bedeutung von $R^{17}$ hat und mit dieser gleich oder verschieden ist,

oder der bis zu 3-fach gleich oder verschieden unterbrochen ist durch Aryliden mit 6 bis 10 Kohlenstoffatomen oder einen cyclischen Rest der Formel

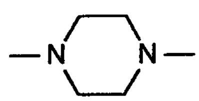 , 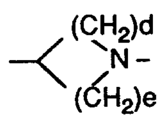 , 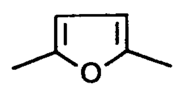 oder 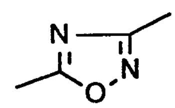

worin

d und e gleich oder verschieden sind und eine Zahl 1 oder 2 bedeuten,
und worin Aryliden und die Heterocyclen ihrerseits durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert sein können,
und wobei der Kohlenwasserstoffrest gegebenenfalls bis zu 3-fach gleich oder verschieden durch Cycloalkyl mit 3 bis 8 Kohlenstoffatomen, Halogen, Nitro, Cyano, Hydroxy, $-O-NO_2$, geradkettiges oder verzweigtes Alkylthio, Alkoxy oder Acyloxy mit jeweils bis zu 8 Kohlenstoffatomen oder durch Aryl, Aryloxy oder Arylthio mit jeweils 6 bis 10 Kohlenstoffatomen oder durch einen 5- bis 7-gliedrigen, gesättigten oder ungesättigen Heterocyclus mit bis zu 3 Heteroatomen aus der Reihe S, N oder O substituiert ist, wobei die Cyclen ihrerseits bis zu 3-fach gleich oder verschieden durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert sein können, oder
der Kohlenwasserstoffrest durch eine Gruppe der Formel $-CO_2-R^{19}$, $-CONR^{20}R^{21}$ oder $-NR^{22}R^{23}$ substituiert ist,
worin

$R^{19}$ die oben angegebene Bedeutung von $R^{17}$ hat und mit dieser gleich oder verschieden ist
und

$R^{20}$, $R^{21}$, $R^{22}$ und $R^{23}$ die oben angegebene Bedeutung von $R^{12}$ und $R^{13}$ haben und mit diesen gleich oder verschieden sind,

$R^{15}$ und $R^{16}$ gleich oder verschieden sind und geradkettiges oder verzweigtes Alkyl mit bis zu 8 Kohlenstoffatomen bedeuten,
oder

$R^{15}$ und $R^{16}$ gemeinsam unter Einbezug der Sauerstoffatome und des Phosphors einen 5- bis 7-gliedrigen, gesättigen Heterocyclus bilden,

und deren Salze,

mit der Maßgabe, daß $R^5$ nicht für unsubstituiertes Alkyl stehen darf, wenn $R^1$ gleichzeitig unsubstituiertes Alkyl bedeutet oder $R^1$ und $R^2$ gemeinsam einen Lactonring bilden,

dadurch gekennzeichnet, daß man im Fall, daß $R^1$ und $R^2$ nicht gemeinsam einen Lactonring bilden,

[A] Verbindungen der allg. Formel (II)

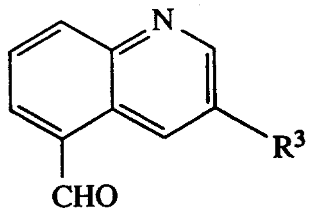

(II)

in welcher

$R^3$ die oben angegebene Bedeutung hat,

zunächst mit Acylderivaten der allgemeinen Formel (III)

$R^5\text{-CO-CH}_2\text{-}R^4$ (III)

in welcher

$R^4$ und $R^5$ die oben angegebene Bedeutung haben,

gegebenenfalls unter Isolierung der entsprechenden Ylidenverbindungen der allgemeinen Formel (IV)

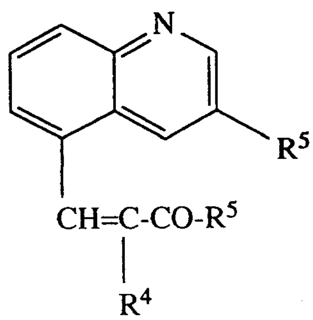

(IV)

in welcher

$R^3$, $R^4$ und $R^5$ die oben angegebene Bedeutung haben,

umsetzt und anschließend entweder mit Verbindungen der Formel (V)

$R^1\text{-CO-CH}_2\text{-}R^2$ (V)

in welcher

$R^1$ und $R^2$ die oben angegebene Bedeutung haben,

in Anwesenheit von Ammoniak oder Ammoniumsalzen, oder direkt mit Aminoderivaten der allgemeinen Formel (VI)

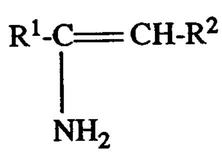

(VI)

in welcher

$R^1$ und $R^2$ die oben angegebene Bedeutung haben,

gegebenenfalls in Gegenwart von inerten organischen Lösemitteln umsetzt,

oder

[B] die Aldehyde der allgemeinen Formel (II) zunächst mit den Verbindungen der allgemeinen Formel (V), gegebenenfalls unter Isolierung der Ylidenverbindungen der allgemeinen Formel (VII)

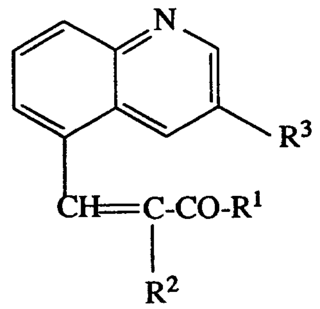

(VII)

in welcher

$R^1$, $R^2$ und $R^3$ die oben angegebene Bedeutung haben,

umsetzt und in einem nächsten Schritt mit den oben aufgeführten Verbindungen der allgemeinen Formel (III) in inerten Lösemitteln, in Anwesenheit von Ammoniak oder Ammoniumsalzen oder direkt mit Enaminocarbonsäurederivaten der allgemeinen Formel (VIII)

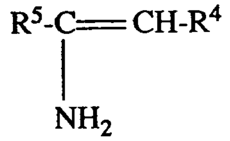

(VIII)

in welcher

$R^4$ und $R^5$ die oben angegebene Bedeutung haben,

umsetzt,

oder im Fall, daß $R^1$ und $R^2$ gemeinsam einen Lactonring bilden,

[C] zunächst nach denen unter [A] und [B] aufgeführten Methoden, Verbindungen der allgemeinen Formel (IX)

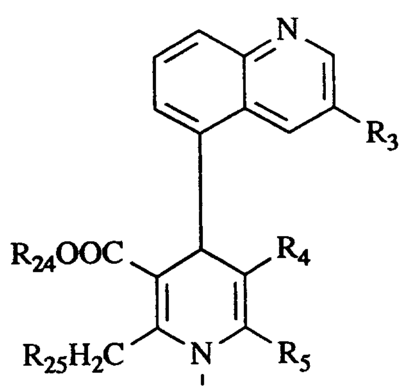

(IX)

in welcher

$R^3$, $R^4$ und $R^5$ die oben angegebene Bedeutung haben,

$R^{24}$ für einen $C_1$-$C_6$-Alkyl-Rest steht

und

$R^{25}$ für eine übliche Abgangsgruppe steht,

herstellt und nach üblichen Verfahren einen säure- oder basekatalysierten Ringschluß anschließt.

**6.** Verbindungen der allgmeinen Formel (I) gemäß Anspruch 1 zur Verwendung bei der Behandlung von Herz-Kreislauferkrankungen.

**7.** Arzneimittel enthaltend mindestens eine Verbindung der allgemeinen Formel (I) gemäß Anspruch 1.

**8.** Verfahren zur Herstellung von Arzneimitteln, dadurch gekennzeichnet, daß man mindestens eine Verbindung der allgemeinen Formel (I) gemäß Anspruch 1 gegebenenfalls mit üblichen Hilfs und Trägerstoffen in eine geeignete Applikationsform überführt.

**9.** Verwendung von Verbindungen der allgemeinen Formel (I) gemäß Anspruch 1 in Arzneimitteln mit positiv ionotroper Wirkung.